# FASCICULE DE BREVET EUROPEEN

(11) **EP 3 295 899 B1**
(45) Date de publication et mention de la délivrance du brevet: **17.04.2019**
(21) Numéro de dépôt: 17191646.3
(22) Date de dépôt: 18.09.2017
(51) Int. Cl.: A61F 2/36

(54) **PARTIE FORMANT TIGE FÉMORALE D'UNE PROTHÈSE DE HANCHE**
TEIL, DER DEN OBERSCHENKELHALS EINER HÜFTPROTHESE BILDET
PART FORMING A FEMORAL STEM OF A HIP PROSTHESIS

(30) Priorité: 20.09.2016 FR 1670539
(43) Date de publication de la demande: 21.03.2018
(73) Titulaire: X.NOV IP, 1882 Luxembourg (LU)
(72) Inventeur: PAILHE, Régis, 38000 Grenoble (FR); SARAGAGLIA, Dominique, 38640 Claix (FR); MERTL, Patrice, 80000 Amiens (FR); GABRION, Antoine, 80090 Amiens (FR); BIEGUN, Frédérique, 2926 Boncourt (CH); BIEGUN, Jean-François, 2926 Boncourt (CH); LOEHLE, Pascal, 2904 Bressaucourt (CH)
(74) Mandataire: Eidelsberg, Olivier Nathan

(56) Documents cités:
- WO-A2-2009/037284
- DE-U1-202004 016 353
- US-A1- 2012 095 568
- US-A1- 2013 030 543

## Description

La présente invention se rapporte à une partie formant tige fémorale d'une prothèse de hanche, qui comporte une partie inférieure distale, notamment de forme sensiblement conique, destinée à être insérée dans la partie diaphysaire du canal médullaire du fémur, une partie intermédiaire destinée à être dans la partie métaphysaire du canal médullaire et une partie supérieure proximale, notamment en forme de cône morse, destinée à coopérer avec une tête sphérique destinée à être reçue dans une cupule cotyloïdienne. La présente invention se rapporte également à une prothèse totale de hanche comportant une partie formant tige fémorale de ce genre.

Classiquement, la partie distale, ou diaphysaire, d'une tige fémorale de ce genre est sensiblement symétrique en coupe transversale dans le plan frontal par rapport à un axe neutre ou axe vertical. Lorsque le chirurgien insère la tige dans le canal médullaire, il le fait par le dessus et, à cet effet, réalise une incision dans la peau du patient au-dessus de la hanche pour permettre le passage de la tige.

On connaît de WO 2009/037284 ou de DE 20 2004 016353 des tiges fémorales de ce genre.

La présente invention vise à surmonter les inconvénients de l'art antérieur en proposant une tige de prothèse de hanche qui limite au maximum l'incision qu'il convient de réaliser dans la peau au-dessus de la hanche pour son insertion dans le canal médullaire.

Suivant l'invention, une tige fémorale d'une prothèse de hanche, comportant une partie inférieure destinée à être ancrée dans la partie diaphysaire du canal médullaire du fémur, une partie intermédiaire destinée à être dans la partie métaphysaire du canal médullaire et une partie supérieure en forme de col prothétique destinée à coopérer, du côté interne de la tige, avec une tête sphérique destinée à être reçue dans une cupule de cotyle d'une prothèse totale de hanche, la partie inférieure ou diaphysaire ayant une face latérale externe s'étendant jusqu'à un point d'extrémité inférieure et comportant, en coupe transversale dans le plan frontal, à partir de la fin de la partie intermédiaire métaphysaire vers le point d'extrémité, un premier tronçon sensiblement rectiligne, est caractérisée en ce que la face latérale externe de la partie inférieure ou diaphysaire comporte un deuxième tronçon s'étendant après le premier tronçon de sorte que la droite s'étendant de la fin du premier tronçon jusqu'au point d'extrémité distale étant inclinée vers l'intérieur par rapport au premier tronçon d'un angle inférieur à 60°, notamment compris entre 15° et 55°, pour former un biseau, notamment un angle compris entre 20 et 50°, de préférence entre 25° et 45°, plus préférentiellement entre 30 et 40°, notamment 35°.

En prévoyant ainsi une partie en biseau au niveau de l'extrémité distale inférieure de la tige, du côté externe, c'est-à-dire du côté opposé au côté duquel s'étend le col prothétique destiné à coopérer avec la cupule, on permet une insertion plus aisée de la tige dans le canal médullaire. Notamment, le dégagement à réaliser par incision de la peau au-dessus de la hanche est moindre que dans le cas des tiges de l'art antérieur. En effet, pour introduire la tige dans le canal, on peut l'introduire non plus parallèlement au canal médullaire, mais inclinée par rapport à ce dernier, la présence de la partie en biseau permettant de faire pivoter la tige lors de son insertion pour l'introduire bien au fond du canal. Dans le même temps, il n'apparaît aucun effet négatif sur la qualité de l'ancrage final de la tige dans le fémur. Grâce à ce système, on peut même prévoir, si on le souhaite, mais sans que cela soit une caractéristique obligatoire de la présente invention, une tige de longueur inférieure à celle des tiges actuelles sans que la qualité de l'ancrage ne soit détériorée de quelque manière que ce soit.

De préférence, le deuxième tronçon est sensiblement rectiligne, notamment a, en section frontale, la forme d'un tronçon elliptique de grand rayon de courbure.

Suivant un mode de réalisation avantageux, le deuxième tronçon, en section frontale, est rectiligne.

De préférence, le deuxième tronçon rectiligne ou sensiblement rectiligne s'étend jusqu'au point d'extrémité distal.

Suivant un mode de réalisation préféré de l'invention, le point d'extrémité distal est décalé par rapport à l'axe neutre vertical, et notamment est décalé du côté intérieur, notamment d'une distance pouvant aller jusqu'à 4 mm.

De préférence, la face externe latérale se poursuit dans la partie supérieure métaphysaire sous la forme d'un troisième tronçon sensiblement rectiligne s'étendant depuis une face supérieure sensiblement horizontale, appelée talon de la tige, jusqu'au premier tronçon, notamment en étant incliné vers l'intérieur par rapport au premier tronçon, notamment d'un angle compris entre 5 et 20°.

Suivant un mode de réalisation préféré de l'invention, les hauteurs mesurées le long de l'axe neutre vertical respectivement des premier et deuxième tronçons h1, h2 sont telles que le rapport de h2 sur la hauteur totale de la tige hors la partie supérieure en forme de col prothétique, notamment la somme de h1, h2 et de la hauteur h3 du troisième tronçon, est inférieur à 0,5, notamment inférieur à 0,25, notamment inférieur à 0,16.

Suivant un mode de réalisation préféré de l'invention, la hauteur de la tige hors la partie supérieure en forme de col prothétique, notamment la somme H= h1+h2+h3, est comprise entre 100 et 150mm en fonction des tailles choisies.

La présente invention se rapporte également à une prothèse totale de hanche comportant une tige suivant l'invention et une cupule de cotyle.

A titre d'exemple, on décrit maintenant un mode de réalisation préféré de l'invention en se reportant aux dessins dans lesquels :
- la figure 1: est une vue en coupe transversale frontale d'un premier mode de réalisation d'une tige de prothèse de hanche suivant l'invention ;
- la figure 2: est une vue du côté externe de la tige de la figure 1 ;
- les figures 3a et 3b: sont des schémas représentant la manière dont sont insérées respectivement une tige de l'art antérieur et la tige de la figure 1 dans le canal médullaire ;
- la figure 4: est une vue en coupe transversale frontale d'un deuxième mode de réalisation d'une tige de prothèse de hanche suivant l'invention ;
- les figures 5a et 5b: sont des schémas représentant la manière dont sont insérées respectivement une tige de l'art antérieur et la tige de la figure 4 dans le canal médullaire ;
- la figure 6: est une vue à plus grande échelle de la partie inférieure de la tige des figures 4 et 5b ; et
- la figure 7: est une vue en coupe à encore plus grande échelle de la partie inférieure de la tige de la figure 6.

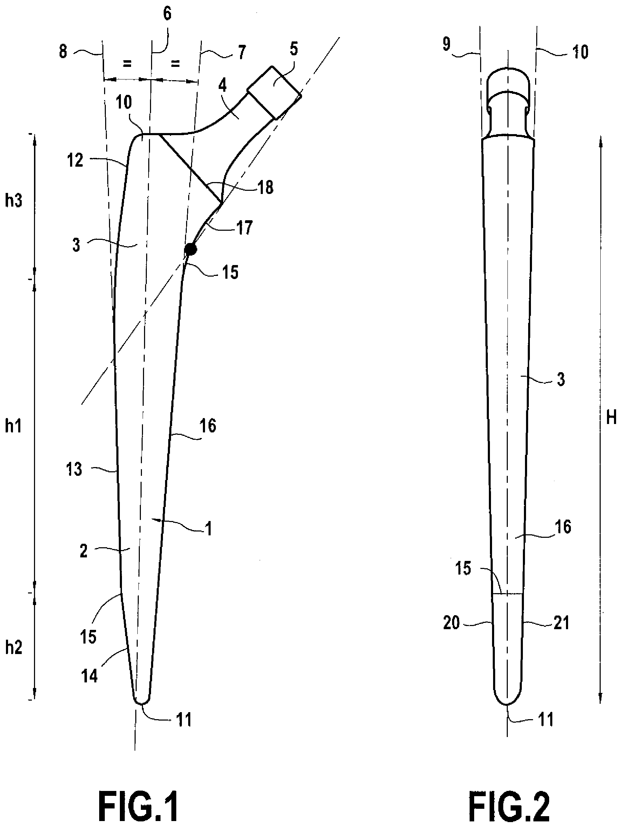

A la figure 1, la tige 1 peut être décomposée en trois parties, à savoir une partie 2 inférieure ou distale, dite partie diaphysaire, poursuivie par une partie 3 intermédiaire, dite métaphysaire, et se terminant au-delà de la partie 3 métaphysaire par une partie 4 dite épiphysaire comportant à son extrémité proximale un cône 5 morse destiné à être reçu dans une tête sphérique destinée à coopérer avec une cupule destinée à être insérée dans la cavité cotyloïdienne de la hanche pour ainsi former une prothèse totale de hanche.

En coupe transversale dans le plan frontal, comme représenté à la figure 1, la face latérale externe (c'est à dire la face destinée à se trouver du côté externe du fémur une fois la tige insérée dans le canal médullaire du fémur) s'étend vers le bas à partir d'une face 10 supérieure sensiblement horizontale et perpendiculaire à l'axe 6 neutre vertical. La face 10 est classiquement appelée le talon 10 de la tige fémorale. La face latérale externe s'étend jusqu'à un point d'extrémité 11 inférieur distal.

Entre le talon 10 et le point d'extrémité 11, la face latérale extérieure est constituée d'une face 12 supérieure sensiblement plane (en forme de segment sensiblement rectiligne en coupe transversale dans le plan frontal), suivie d'une face 13 intermédiaire sensiblement plane (également en forme de segment sensiblement rectiligne en coupe transversale dans le plan frontal), inclinée par rapport à la première face 12, notamment d'un angle compris entre 5° et 20°, et terminée par une face 14 inférieure sensiblement plane(également en forme de segment sensiblement rectiligne en coupe transversale dans le plan frontal).

En coupe frontale, le tronçon 14 s'étend à partir d'un point 15 de rupture de pente par rapport au tronçon 13 intermédiaire jusqu'au point 11 d'extrémité. Le tronçon 14 est incliné par rapport au tronçon 13 d'un angle compris entre 20 et 50°, notamment entre 30 et 40°, notamment est égal à 35°.

Entre le point 11 sommet et le cône morse, du côté intérieur, la face intérieure de la prothèse comporte une face 16 sensiblement plane (en forme de segment sensiblement rectiligne en coupe transversale dans le plan frontal) suivie d'une face 17 incurvée allant jusqu'au début de la partie épiphysaire, cette dernière étant délimitée par la surface 18 épiphysaire.

Vue de côté (correspondant à une vue dans le plan sagittal), comme représentée à la figure 2, on voit que la prothèse est de forme conique, les deux faces 9 et 10 planes ou sensiblement planes étant inclinées l'une par rapport à l'autre d'un angle compris entre 2 et 10°. De même, la face 13 intermédiaire et la face 16 rectiligne extérieure sont disposées symétriquement par rapport à l'axe neutre 6, en étant inclinées l'une par rapport à l'autre d'un angle compris entre 2 et 10°. Ainsi, la partie de la tige s'étendant entre les faces latérales externe et interne 13 et 16 et les faces antérieure et postérieure 9 et 10 forment un tronc de cône. De même, la partie de la tige en dessous de la rupture de pente 15 définit un cône jusqu'au point 11. Cependant l'axe de ce cône inférieur est incliné par rapport à l'axe 6, notamment d'un angle correspondant à la rupture de pente.

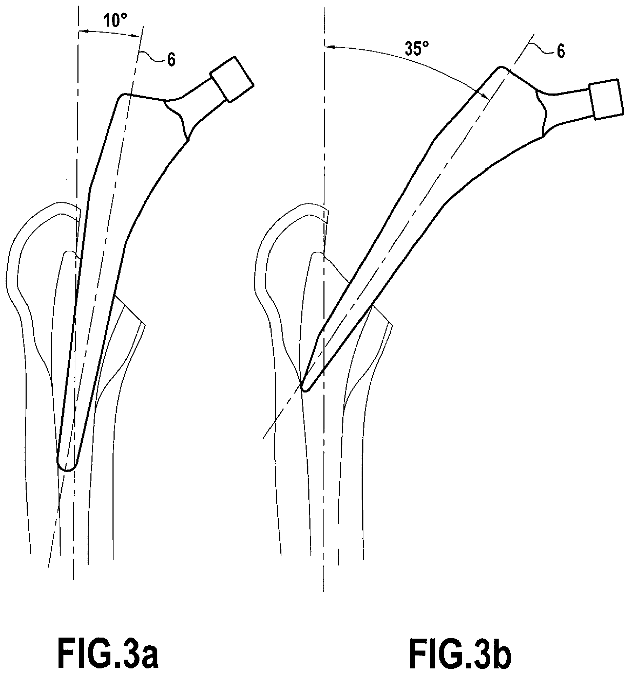

En prévoyant ainsi une face 14 inclinée par rapport à la face 13 intermédiaire, on améliore fortement la capacité d'introduction de la tige dans le canal médullaire, notamment on diminue fortement la quantité de peau à inciser au-dessus de la prothèse lors de l'insertion, l'insertion étant fortement facilitée. En effet, dans l'art antérieur, il était nécessaire d'insérer la prothèse quasiment verticalement dans le canal, et par conséquent d'inciser préalablement sur une grande distance la peau au-dessus de la prothèse. Suivant l'invention, on peut l'insérer alors qu'elle est inclinée par rapport au canal médullaire (voir figure 3b), notamment de l'angle correspondant à l'angle entre la face 14 et la face 13. Lorsque la face 14 bute contre la paroi externe du canal médullaire, on fait alors pivoter la tige dans le sens de la flèche de la figure 3b pour poursuivre l'insertion jusqu'au fond du canal.

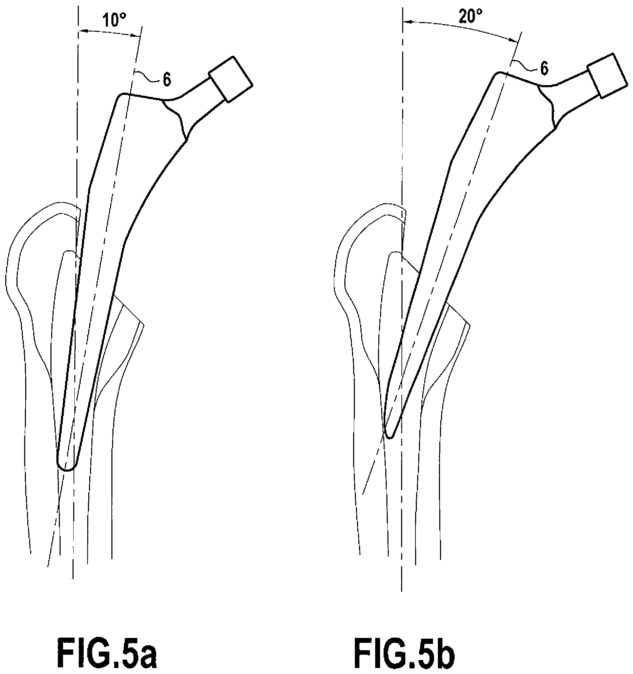

Dans l'art antérieur, pour atteindre un point le plus bas possible du canal médullaire avec la tige, il était nécessaire de prévoir une tige la plus longue possible (voir les figures 3a ou 5a). Grâce à ce système permettant le pivotement lors de l'insertion, on obtient un ancrage aussi bon que celui que l'on obtient avec les prothèses de l'art antérieur, mais ce avec une tige moins longue et/ou moins volumineuse, donc plus facile à manipuler pour le chirurgien.

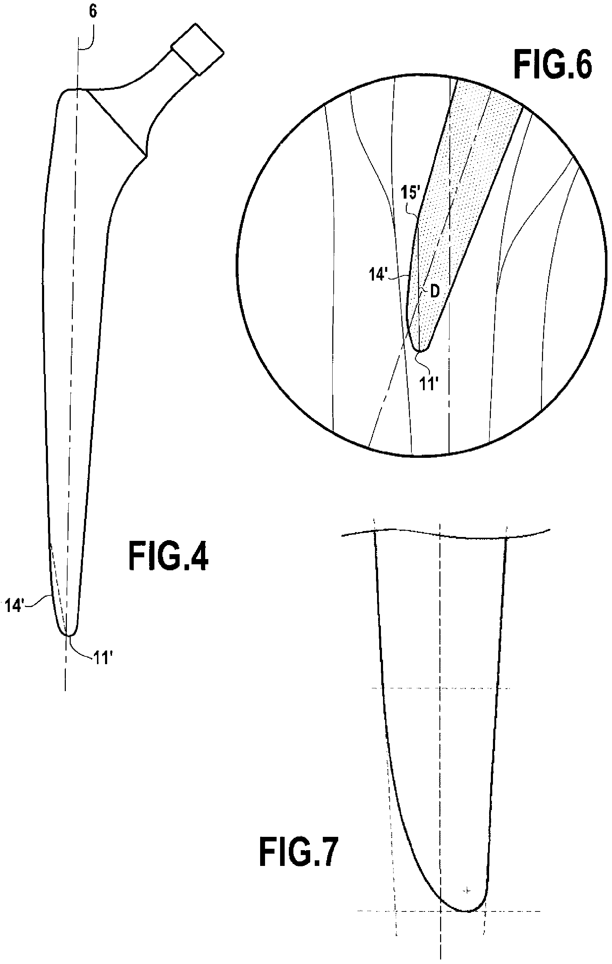

Aux figures 4, 5b, 6 et 7, il est représentée un tige suivant un autre mode de réalisation.

Le troisième tronçon 14' n'est que sensiblement rectiligne, en étant ici de forme elliptique ou ovale. Cependant, la droite (D) allant du point 15' de rupture de pente jusqu'au point 11' d'extrémité distale est inclinée par rapport au tronçon 13' suivant un angle ayant les même caractéristiques que celui formé entre les tronçons 13 et 14 du premier mode de réalisation, notamment est compris entre 20 et 50°, notamment entre 30 et 40°, notamment est égal à 35°. En particulier, la tangente au point 15' du tronçon 14' du côté inférieur ou distal fait un angle par rapport au tronçon 13' qui est inférieur à 30°, notamment inférieur à 20°, par exemple inférieur à 10°.

La forme elliptique du tronçon 14' est particulièrement avantageuse pour améliorer encore plus la solidité de l'ancrage de la tige dans le canal.

Comme on le voit dans les deux modes de réalisation, le point 11, 11' d'extrémité est décalé vers le côté intérieur par rapport à l'axe 6 neutre ou vertical. Ce décalage peut notamment aller jusqu'à 4mm.

De préférence, la hauteur totale H de la tige du côté externe, à savoir du talon jusqu'à la pointe 11, 11', mesurée le long de l'axe neutre, peut être inférieure à 150mm.

En outre, il est favorable que h3 soit compris entre 30mm et 50mm, tandis que h2 soit compris entre 45mm et 70mm et h1 entre 20mm et 30mm. En particulier, le rapport de h3 sur h1+h2 est compris entre 10% et 35%, notamment entre 15% et 25%.

## Revendications

1. Tige (1) fémorale d'une prothèse de hanche, ayant un axe (6) neutre vertical et comportant une partie (2) inférieure destinée à être ancrée dans la partie diaphysaire du canal médullaire du fémur, une partie (3) intermédiaire destinée à être dans la partie métaphysaire du canal médullaire et une partie supérieure en forme de col prothétique destinée à coopérer, du côté interne de la tige, avec une tête sphérique destinée à être reçue dans une cupule de cotyle d'une prothèse totale de hanche, la partie inférieure ou diaphysaire ayant une face latérale externe s'étendant jusqu'à un point (11 ; 11') d'extrémité inférieure et comportant, en coupe transversale dans le plan frontal, à partir de la fin de la partie intermédiaire métaphysaire vers le point (11 ; 11') d'extrémité, un premier tronçon (13 ; 13') sensiblement rectiligne, la face latérale externe de la partie inférieure ou diaphysaire comportant un deuxième tronçon (14 ; 14') s'étendant après le premier tronçon (13) de sorte que la droite s'étendant de la fin du premier tronçon (13) jusqu'au point (11 ; 11') d'extrémité distale est inclinée vers l'intérieur par rapport au premier tronçon (13 ; 13') d'un angle inférieur à 60°, notamment compris entre 15° et 55°, pour former un biseau, notamment un angle compris entre 20 et 50°, de préférence entre 25° et 45°, plus préférentiellement entre 30 et 40°, notamment 35°, **caractérisé en ce que** le point (11 ; 11') d'extrémité distal est décalé par rapport à l'axe (6) neutre vertical.

2. Tige suivant la revendication 1, **caractérisée en ce que** le deuxième tronçon (14') est sensiblement rectiligne.

3. Tige suivant la revendication 1 ou 2, **caractérisée en ce que** le deuxième tronçon (14) est rectiligne.

4. Tige suivant la revendication 1 ou 2, **caractérisée en ce que** le deuxième tronçon (14') est en forme de secteur elliptique.

5. Tige suivant l'une des revendications 1 à 4, **caractérisée en ce que** le deuxième tronçon (14 ; 14') s'étend jusqu'au point d'extrémité distal.

6. Tige suivant la revendication 5, **caractérisée en ce que** le point (11 ; 11') d'extrémité distal est décalé par rapport à l'axe (6) neutre vertical du côté intérieur.

7. Tige suivant la revendication 5 ou 6, **caractérisée en ce que** le point (11 ; 11') d'extrémité distal est décalé par rapport à l'axe (6) neutre vertical d'une distance pouvant aller jusqu'à 4 mm.

8. Tige suivant l'une des revendications 1 à 7, **caractérisée en ce que** la face externe latérale se poursuit dans la partie supérieure métaphysaire sous la forme d'un troisième tronçon (12) sensiblement rectiligne s'étendant depuis une face supérieure sensiblement horizontale, appelée talon de la tige, jusqu'au premier tronçon (13 ; 13'), notamment en étant incliné vers l'intérieur par rapport au premier tronçon, notamment d'un angle compris entre 5° et 20°.

9. Tige suivant l'une des revendications 1 à 8, **caractérisée en ce que**, les hauteurs mesurées le long de l'axe neutre vertical respectivement des premier et deuxième tronçons h1, h2 sont telles que le rapport de h2 sur la hauteur totale de la tige hors la partie supérieure en forme de col prothétique, notamment la somme de h1, h2 et de la hauteur h3 du troisième tronçon, est inférieur à 0,5, notamment inférieur à 0,25, notamment inférieur à 0,16.

10. Tige suivant l'une des revendications 1 à 9, **caractérisée en ce que** la hauteur de la tige hors la partie supérieure en forme de col prothétique, notamment la somme H = h1+h2+h3, est inférieure à 150 mm.

11. Prothèse totale de hanche comportant une tige suivant l'une des revendications précédentes et une cupule de cotyle.

## Patentansprüche

1. Femurstiel (1) einer Hüftprothese, der eine neutrale Vertikalachse (6) aufweist und einen unteren Teil (2), welcher dazu bestimmt ist, in dem diaphysären Teil des Markkanals des Oberschenkels verankert zu werden, einen Zwischenteil (3), der dazu bestimmt ist, in dem metaphysären Teil des Markkanals zu liegen, und einen oberen Teil in Form eines Prothesenhalses, der dazu bestimmt ist, auf der inneren Seite des Stiels mit einem Kugelkopf zusammenzuwirken, der dazu bestimmt ist, in einer Schale einer Gelenkpfanne einer Hüfttotalendoprothese aufgenommen zu werden, umfasst, wobei der untere oder diaphysäre Teil eine äußere Seitenfläche aufweist, die sich bis zu einem unteren Endpunkt (11; 11') erstreckt und im Querschnitt in der Frontalebene von dem Ende des metaphysären Zwischenteils bis zu dem Endpunkt (11; 11') einen im Wesentlichen geradlinigen ersten Abschnitt (13; 13') der äußeren Seitenfläche des unteren oder diaphysären Teils umfasst, einen zweiten Abschnitt (14; 14') umfasst, der sich hinter dem ersten Abschnitt (13) so erstreckt, dass die von dem Ende des ersten Abschnitts (13) bis zu dem distalen Endpunkt (11; 11') verlaufende Gerade bezüglich des ersten Abschnitts (13; 13') in einem Winkel von unter 60°, insbesondere zwischen 15° und 55°, nach innen geneigt ist, um eine Abschrägung, insbesondere einen Winkel zwischen 20 und 50°, vorzugsweise zwischen 25° und 45°, besonders bevorzugt zwischen 30 und 40°, insbesondere 35°, zu bilden,
**dadurch gekennzeichnet, dass** der distale Endpunkt (11; 11') bezüglich der neutralen Vertikalachse (6) versetzt ist.

2. Stiel nach Anspruch 1, **dadurch gekennzeichnet, dass** der zweite Abschnitt (14) im Wesentlichen geradlinig ist.

3. Stiel nach Anspruch 1 oder 2, **dadurch gekennzeichnet, dass** der zweite Abschnitt (14) geradlinig ist.

4. Stiel nach Anspruch 1 oder 2, **dadurch gekennzeichnet, dass** der zweite Abschnitt (14') in Form eines elliptischen Sektors vorliegt.

5. Stiel nach einem der Ansprüche 1 bis 4, **dadurch gekennzeichnet, dass** sich der zweite Abschnitt (14; 14') bis zu dem distalen Endpunkt erstreckt.

6. Stiel nach Anspruch 5, **dadurch gekennzeichnet, dass** der distale Endpunkt (11; 11') bezüglich der neutralen Vertikalachse (6) von der Innenseite versetzt ist.

7. Stiel nach Anspruch 5 oder 6, **dadurch gekennzeichnet, dass** der distale Endpunkt (11; 11') bezüglich der neutralen Vertikalachse (6) um eine Strecke, die sich bis auf 4 mm belaufen kann, versetzt ist.

8. Stiel nach einem der Ansprüche 1 bis 7, **dadurch gekennzeichnet, dass** die äußere Seitenfläche im metaphysären oberen Teil in Form eines dritten Abschnitts (12) weiterverläuft, der sich von einer im Wesentlichen horizontalen oberen Fläche, die Stielferse genannt wird, im Wesentlichen geradlinig bis zu dem ersten Abschnitt (13; 13') erstreckt, wobei sie insbesondere bezüglich des ersten Abschnitts nach innen, insbesondere in einem Winkel zwischen 5° und 20°, geneigt ist.

9. Stiel nach einem der Ansprüche 1 bis 8, **dadurch gekennzeichnet, dass** die entlang der neutralen Vertikalachse gemessenen Höhen des ersten bzw. zweiten Abschnitts h1, h2 derart sind, dass das Verhältnis von h2 zu der Gesamthöhe des Stiels außerhalb des oberen Teils in Form eines Prothesenhalses, insbesondere die Summe von h1, h2 und der Höhe h3 des dritten Abschnitts kleiner als 0,5, insbesondere kleiner als 0,25, insbesondere kleiner als 0,16, ist.

10. Stiel nach einem der Ansprüche 1 bis 9, **dadurch gekennzeichnet, dass** die Höhe des Stiels außerhalb des oberen Abschnitts in Form eines Prothesenhalses, insbesondere die Summe H = h1+h2+h3, kleiner als 150 mm ist.

11. Hüfttotalendoprothese, die einen Stiel nach einem der vorhergehenden Ansprüche und eine Schale einer Gelenkpfanne umfasst.

## Claims

1. A femoral shaft (1) of a hip prosthesis, having a vertical neutral axis (6) and comprising a lower portion (2) intended to be fixed in the diaphyseal portion of the medullary cavity of the femur, an intermediate portion (3) intended to be in the metaphyseal portion of the medullary cavity and an upper portion in the form of a prosthetic collar intended to cooperate, from inside the shaft, with a spherical head intended to be received in an acetabular cup of a total hip prosthesis, the inner or diaphyseal portion having an outer lateral face extending as far as a tip (11; 11') of the inner end and comprising, in transverse section in the frontal plane, from the end of the metaphyseal intermediate portion to the tip (11; 11') of the end, a first, substantially rectilinear section (13; 13'), the lateral external face of the inner or diaphyseal portion comprising a second section (14; 14') extending after the first section (13) such that the straight line extending from the end of the first section (13) as far as the tip (11; 11') of the distal end is inclined to the inside in respect of first section (13; 13') at an angle of less than 60°, specifically between 15° and 55°, to form a bevel, specifically an angle between 20 and 50°, preferably between 25° and 45°, more preferably between 30 and 40°, specifically 35°, **characterised in that** the tip (11; 11') of the distal end is offset in respect of the vertical neutral axis (6).

2. The shaft according to claim 1, **characterised in that** the second section (14') is substantially rectilinear.

3. The shaft according to claim 1 or 2, **characterised in that** the second section (14) is rectilinear.

4. The shaft according to claim 1 or 2, **characterised in that** the second section (14') is in the form of an elliptical segment.

5. The shaft according to one of claims 1 to 4, **characterised in that** the second section (14; 14') extends as far as the end of the distal end.

6. The shaft according to claim 5, **characterised in that** the tip (11; 11') of the distal end is offset in respect of the vertical neutral axis (6) of the inner side.

7. The shaft according to claim 5 or 6, **characterised in that** the tip (11; 11') of the distal end is offset by a distance of up to 4mm relative to the vertical neutral axis (6).

8. The shaft according to one of claims 1 to 7, **characterised in that** the outer lateral face follows into the upper metaphyseal portion in the form of a third, substantially rectilinear section (12) extending from a substantially horizontal upper face, called heel of the shaft, as far as the first section (13; 13'), specifically by being inclined inwardly in respect of the first section, specifically at an angle between 5° and 20°.

9. The shaft according to one of claims 1 to 8, **characterised in that** the heights measured along the vertical neutral axis, respectively of the first and second sections h1, h2, are such that the ratio of h2 on the total height of the shaft not including the upper portion in the form of a prosthetic collar, specifically the sum of h1, h2 and height h3 of the third section, is less than 0.5, specifically less than 0.25, specifically less than 0.16.

10. The shaft according to one of claims 1 to 9, **characterised in that** the height of the shaft, not including the upper portion in the form of a prosthetic collar, specifically the sum H = h1+h2+h3, is less than 150 mm.

11. A total hip prosthesis comprising a shaft according to one of the preceding claims and an acetabular cup.
